(19) Europäisches Patentamt
European Patent Office
Office européen des brevets

(11) Publication number: **0 257 421 B1**

(12) **EUROPEAN PATENT SPECIFICATION**

(45) Date of publication of patent specification: **16.09.92**

(21) Application number: **87111536.6**

(22) Date of filing: **10.08.87**

(51) Int. Cl.5: **C12P 21/00**, C07K 15/00, C07K 17/02, C12N 15/00, G01N 33/577, G01N 33/68, C07K 7/00, C07K 9/00, //C12N5/00,(C12P21/00, C12R1:91)

(54) **Antibodies for use in determining human glycoalbumin.**

(30) Priority: **22.08.86 US 899456**
**02.06.87 US 54131**

(43) Date of publication of application:
**02.03.88 Bulletin 88/09**

(45) Publication of the grant of the patent:
**16.09.92 Bulletin 92/38**

(84) Designated Contracting States:
**AT BE CH DE ES FR GB IT LI LU NL**

(56) References cited:
**EP-A- 0 111 211**
**EP-A- 0 230 934**
**WO-A-88/00346**
**US-A- 4 478 744**

**THE JOURNAL OF BIOLOGICAL CHEMISTRY, vol. 258, no. 10, 25th May 1983, pages 6142-6146, US; R.L. GARLICK et al.: "The principal site of nonenzymatic glycosylation of human serum albumin in vivo"**

(73) Proprietor: **MILES INC.**
**One Mellon Center 500 Grant Str.**
**Pittsburgh, PA 15219-2502(US)**

(72) Inventor: **Knowles, William J.**
**85 Fawnbrook Circle**
**Madison, CT 06443(US)**
Inventor: **Marchesi, Vincent T.**
**179 Prospect Avenue**
**Guilford, CT 06437(US)**

(74) Representative: **Dänner, Klaus, Dr. et al**
**c/o Bayer AG Konzernverwaltung RP Patente Konzern**
**W-5090 Leverkusen 1 Bayerwerk(DE)**

**EP 0 257 421 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

CHEMICAL ABSTRACTS, vol. 103, no. 21, 25th November 1985, page 356, abstract no. 174781r, Columbus, Ohio, US; S. TANEDA et al.: "Measurement of nonenzymatically glucosylated proteins by radioimmunoassay", & TONYOBYO (TOKYO) 1985, 28(5), 695-8

CHEMICAL ABSTRACTS, vol. 99, no. 25, 19th December 1983, abstract no. 210871e, Columbus, Ohio, US; L.K. CURTISS et al.: "A novel method for generating region-specific monoclonal antibodies to modified proteins. Application to the identification of human glucosylated low density lipoproteins", & J. CLIN. INVEST. 1983, 72(4), 1427-38

## Description

### BACKGROUND OF THE INVENTION

This invention relates to the determination of the glycosylated form of albumin, herein referred to as glycoalbumin, in human blood samples. The determination of the extent of glycosylation of albumin in an individual's blood provides a useful index of glucose level control in diabetics. In particular, the present invention concerns the preparation of monoclonal antibodies which recognize specifically the glycosylated lysine residue at position 525 in human albumin.

Albumin is the major serum protein of blood and has a half life in circulation of 10 days. A non-enzymatic glycosylation reaction results in the covalent coupling of glucose to a small percentage of albumin molecules in all individuals. Since the rate of non-enzymatic glycosylation is dependent upon the circulating level of glucose, diabetics having a higher average level of blood glucose, have an increase in glycosylated albumin. The severity of the diabetic condition is therefore reflected in the percentage of glycosylated albumin.

An analogous reaction occurs between glucose and hemoglobin producing hemoglobin Alc plus other glycosylated hemoglobins. Hemoglobin has a life of 120 days, therefore determination of glycosylated hemoglobin values reflects the average circulating glucose level for that period, whereas a glycoalbumin determination will represent an average circulating glucose level of 10 days. The importance of glycosylated hemoglobin values have been widely accepted as being clinically important for accurate assessment of the diabetic condition. Assays for glycosylated hemoglobin were relatively easy to develop because the hemoglobin molecule is colored and therefore is easy to quantitate using inexpensive spectrophotometers. Albumin is colorless and methods for quantitation of glycoalbumin require that the carbohydrate be derivatized to a colored product or that the protein portion of glycoalbumin be reacted to produce a colored product. For this reason there are no glycoalbumin assays in large scale use in clinical labs at the present time.

A number of proposed glycoalbumin assays are known from the literature. Principal among these are those based on boronate chromatography and thiobarbituric assays. The boronate chromatography method includes the colorimetric determination of bound protein, e.g., bound to Glycogel of Pierce Chemical Co. In this assay, serum is applied to a boronate affinity column wherein all cis-diol containing substances (e.g., glycoalbumin and other glycoproteins) are bound. These substances are then eluted and both bound and eluate fractions quantitated after adding a dye that reacts with proteins producing a colored product. The major disadvantages of this method are that many non-albumin proteins in serum are glycoproteins (e.g., immunoglobulins) and are therefore bound and measured in the boronate chromatography assay; the column procedure has multiple steps for separation and analysis and is not easily automated; and there is also data suggesting that glucose interfers with boronate binding. In the thiobarbituric assay, the ketoamine-protein adduct is converted to 5-hydroxymethylfurfural by hydrolysis with oxalic acid yielding a colored product. The major disadvantages here are that hydrolysis requires 2-4 hours at 100°C or higher; background color must be corrected; and at the present time, standards or calibrators are not available.

The reaction of glucose with albumin involves (a) the formation of a Schiff's base between C-1 of the glucose with an amino group of albumin and (b) an Amadori rearrangement producing a 1-deoxyfructosyl carbohydrate covalently coupled to the nitrogen of the amino group. The albumin molecule has 60 potential sites (amino groups) for non-enzymatic glycosylation. This is comprised of 59 episilon amino groups of lysine residues and one alpha amino group on the N-terminus of the protein. Of the 60 potential sites only one lysine is known to be glycosylated in the native molecule; however, other lysines may be glycosylated to various extents. The known lysine has been identified as lysine 525 (the 525th amino acid counting from the N-terminus of the protein - Garlick et al, J. Biol. Chem. 258: 6142 (1983)) and the position has been confirmed herein. The reason for the specific glycosylation of this lysine and the rapid rate of glycosylation of albumin is not entirely clear. The specificity for lysine 525 is likely to be (a) the proximity to an adjacent lysine at position 524 thereby lowering the pKa of the ε-amino group of lysine 525 making it more reactive in the glycosylation reaction, (b) the exposure of the lysine 525 side chain to the aqueous exterior of the albumin molecule, and/or (c) the 3-dimensional structure of the albumin molecule that by an unknown mechanism increases the reactivity of lysine 525 for the glycosylation reaction.

Monoclonal antibodies have been shown to have a precise specificity for binding to a variety of organic compounds, including synthetic peptides. However, despite the availability of this technique and the recognized need for an immunoassay for glycoalbumin, an approach to obtaining antibodies useful for the determination of glycoalbumin has not been reported.

The following definitions will be used herein with respect to amino acid units in peptides.

## Definitions

| Amino Acid | Abbreviation |
|---|---|
| Arginine | Arg |
| Aspartic Acid | Asp |
| Glutamic Acid | Glu |
| Lysine | Lys |
| Serine | Ser |
| Asparagine | Asn |
| Glutamine | Gln |
| Glycine | Gly |
| Proline | Pro |
| Threonine | Thr |
| Alanine | Ala |
| Histidine | His |
| Cysteine | Cys |
| Methionine | Met |
| Valine | Val |
| Isoleucine | Ile |
| Leucine | Leu |
| Tyrosine | Tyr |
| Phenylalanine | Phe |
| Tryptophan | Trp |
| Alpha-Aminobutyric Acid | Aba |

## SUMMARY OF THE INVENTION

It is therefore an object of the present invention to provide antibodies specific for binding to glycoalbumin which can serve as the basis of an immunoassay to determine glycoalbumin in human blood samples. There is a well recognized but unresolved need for such an immunoassay which would provide a relatively simple and reliable means for assessing the diabetic condition.

It is particularly an object of the present invention to raise monoclonal antibodies that bind specifically to human albumin that is glycosylated at the lysine residue at position 525, i.e., the 525th amino acid in the peptide chain counting from the end having the free N-terminal amino group. As used herein, a glycosylated amino acid refers to an amino acid having an amino group that has been modified with a 1-deoxyfructosyl residue by non-enzymatic reaction with glucose.

The present invention accomplishes these and other objectives and advantages by providing a method for obtaining somatic cell hybridomas that secrete monoclonal antibodies having the desired glycoablumin specificity. Such hybridomas are formed from conventional fusions of myeloma cells with lymphocytes from an animal, preferably a mouse, that has been immunized with an immunogen comprising an appropriate glycosylated peptide chemically linked to an immunogenic carrier material. The glycosylated peptide in the immunogen is obtained synthetically or by proteolysis and comprises first, a lysine residue whose -amino group is nonenzymatically glycosylated, and second, at least one other amino acid unit in a position corresponding to the peptide sequence of human albumin adjacent to the lysine residue at position 525.

Accordingly, the monoclonal antibody of the present invention binds specifically to a glycosylated

peptide residue of the formula:

$$\begin{array}{c} \text{Glyco-(NH)} \\ | \\ \text{—}AA_1\text{-Lys-}AA_2\text{—} \end{array}$$

wherein Glyco-(NH) represents a nonenzymatically glycosylated ε-amino group in the lysine residue, and one or both of $AA_1$ and $AA_2$ is a sequence of amino acids, preferably containing between 1 and 12 amino acids, wherein at least one, and preferably all, of the amino acid units is in a position corresponding to the peptide sequence of human albumin adjacent to lysine 525, and if only one of $AA_1$ and $AA_2$ is such a sequence, then the other is a bond, a terminal amino or carboxyl group, or additional amino acid residues.

In addition to the above described monoclonal antibodies, including fragments thereof which comprise an antibody ccmbining site, the hybridoma cell lines that secrete such antibody, the method for producing monoclonal antibodies from such cell lines, and the method for obtaining the cell lines and the immunogens used in such process, the present invention also provides an immunoassay method for determining glycoalbumin in a human blood sample such as whole blood, serum, or plasma. In the method, the blood sample is contacted with the monoclonal antibody or fragment thereof of the present invention. Where necessary or desirable, the blood sample is first treated to denature or otherwise expose the epitope at lysine 525 in a significant amount of any glycoalbumin present in the sample. Thereafter, the binding of the antibody reagent to glycoalbumin from the sample is determined following any conventional immunoassay protocol as a function of the amount of glycoalbumin in the sample tested The present invention also provides new and useful peptides and glycosylated forms thereof prepared synthetically or by proteolysis of glycoalbumin or non-glycosylated albumin which can serve as the peptide residue in the immunogen or which are precursors thereof

## BRIEF DESCRIPTION OF THE DRAWINGS

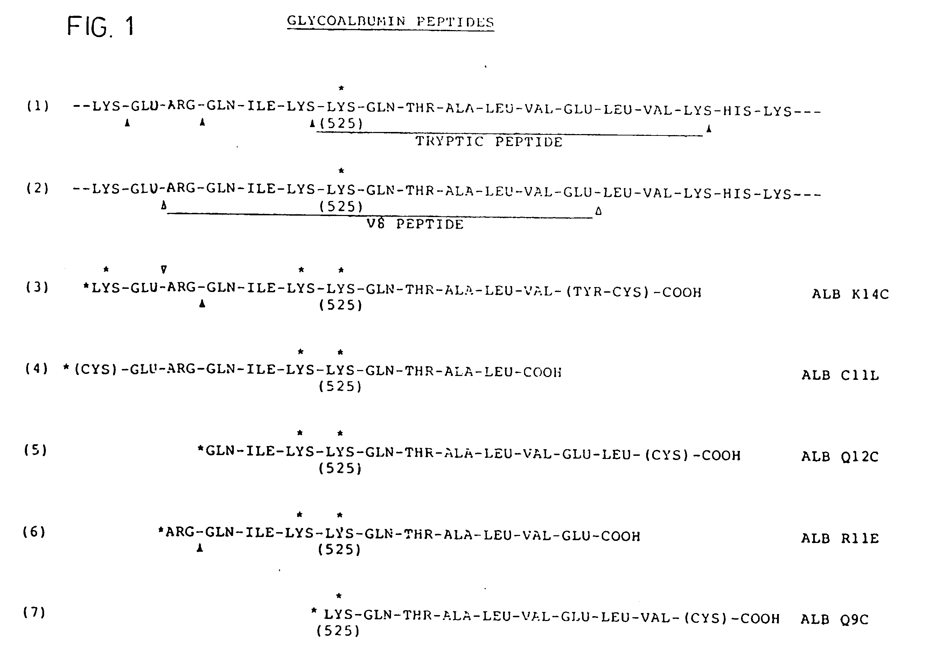

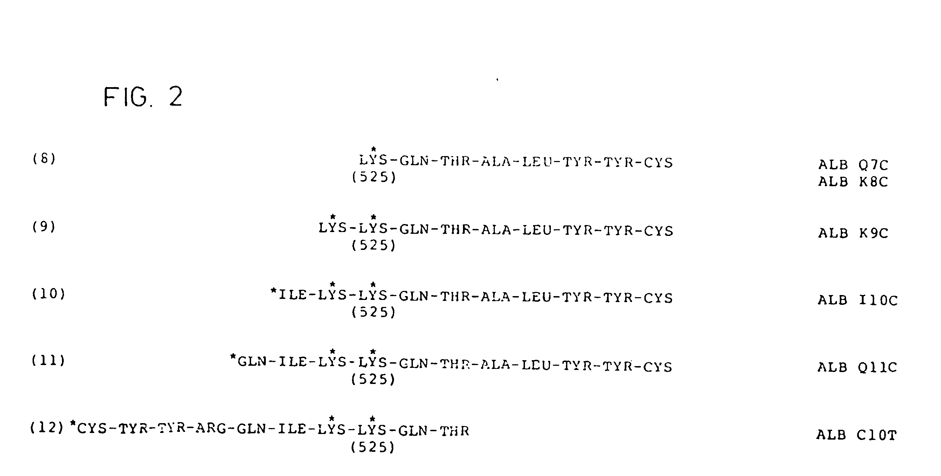

Figs. 1 and 2 of the drawings illustrate some of the preferred glycosylated peptide fragments or residues that can be linked to a conventional immunogenic carrier material to form an immunogen useful in the present invention. Structures (1) and (2) show the partial sequence of glycoalbumin in the region of lysine 525 and the sites of cleavage for the proteolytic enzymes trypsin (solid triangles) and V8 protease (open triangles), respectively. The asterisk above lysine 525 indicates the site of in vivo glycosylation. Structures (3) through (12) show peptide fragments that can be prepared synthetically. The asterisks other than at lysine 525 indicate sites of potentially additional glycosylation during in vitro synthesis. The sequences are shown in the drawing, as well as throughout this description, from N-terminus on the left to C-terminus on the right. Further details and explanations are given in the Examples below.

## DESCRIPTION OF THE PREFERRED EMBODIMENTS

The monoclonal antibody of the present invention is principally characterized by its specificity for binding the glucosylated peptide sequence in the region of lysine 525 in human albumin. This glycosylated residue is the distinguishing structural feature of glycoalbumin. An antibody of the present invention requires an epitope or determinant site comprising minimally the 1-deoxyfructosyl modified lysine unit, formed upon Amadori rearrangement of the reaction product between glucose and the ε-amino group in lysine, and a peptide sequence extending therefrom comprising at least one of the amino acid units in the position corresponding to the glycoalbumin sequence adjacent to lysine 525. The other amino acid units in the peptide sequence characterizing the epitope may be the same or different as those appearing in the native glycoalbumin sequence. In this way, the epitope is characterized as being comprised of a carbohydrate and peptide sequence to which the antibody binds and is unique to the glycosylated lysine 525 sequence in glycoalbumin. Preferably the antibody will specifically bind a glycosylated peptide residue of the formula:

$$\begin{array}{c}\text{Glyco-(NH)} \\ | \\ \text{—}AA_1\text{-Lys-}AA_2\text{—}\end{array} \qquad (A)$$

wherein Glyco-(NH), $AA_1$ and $AA_2$ are as defined above. It is minimally required that at least one of the amino acid sequences $AA_1$ and $AA_2$ comprise an amino acid in a position corresponding to the sequence in glycoalbumin around lysine 525 in order that the antibody binding be specific for glycosylation at lysine 525 rather than any other glycosylated lysine unit in glycoalbumin or any other protein or peptide that might appear in the test sample. Preferably one or both of $AA_1$ and $AA_2$ is a sequence of from 1 to 12 amino acids that correspond exactly to the peptide sequence adjacent to the lysine residue at position 525 in glycoalbumin.

The sequence of glycoalbumin including 12 amino acids on either side of lysine 525 is as follows (in the direction of N-terminus to C-terminus):

**-- Ile-Cys-Thr-Leu-Ser-Glu-Lys-**
**-Glu-Arg-Gln-Ile-Lys-Lys(525)-**
**-Gln-Thr-Ala-Leu-Val-Glu-Leu-**
**-Val-Lys-His-Lys-Pro--**

Most preferably the monoclonal antibody of the present invention will bind specifically to peptide residues of formula (A) above wherein $AA_1$ and $AA_2$ are selected from the following:

**$AA_1$: -Lys-Glu-Arg-Gln-Ile-Lys-,**
**-Glu-Arg-Gln-Ile-Lys-,**
**-Arg-Gln-Ile-Lys-,**
**-Gln-Ile,Lys-,**
**-Ile-Lys-,**
**Lys- or a bond, and**
**$AA_2$: -Gln-Thr-Ala-Leu-Val-Glu-**
**-Gln-Thr-Ala-Leu-Val-**
**-Gln-Thr-Ala-Leu-**
**-Gln-Thr-Ala-**
**-Gln-Thr-**
**-Gln-, or a bond.**

In accordance with the present invention, hybridoma cell lines are raised to produce antibodies only against the glycosylated lysine 525 portion of the albumin molecule rather than to the entire protein and such cell lines and their antibodies are screened to identify and isolate those monoclonal antibodies which will thereafter react selectively with the glycosylated lysine 525 epitope.

To produce such antibodies, a fragment of the protein chain, corresponding to the naturally occurring glycosylated peptide sequence, is coupled to a carrier and injected into a laboratory animal to elicit an immune response. Lymphocytes such as spleen cells from the immunized animal are fused with myeloma cells to produce hybridomas which are cultured and screened for production of monoclonal antibodies. The monoclonal antibodies are screened for those selective to the glycosylated peptide epitope and the particular cell line is cloned for use in producing further quantities of the monoclonal antibody. Reviews of such monoclonal antibody techniques are found in Lymphocyte Hybridomas, Melchers et al, Springer-Verlag (New York 1978), Nature 266:495 (1977), Science 208:692 (1980), and Methods in Enzymology 73-(Part B): 3-46 (1981).

To prepare a suitable immunogen for injection into the laboratory animal, e.g., BALB/c mice, rats or the like, a glycosylated albumin fragment must be either produced and isolated from naturally occurring human albumin or glycoalbumin or be chemically synthesized and purified. The glycosylated peptide fragment useful in the present invention, and its non-glycosylated forms or precursors, is of the formula:

$$(NH_2)(Cys)_q-(Tyr)_r-AA_1-\overset{(QNH)}{\overset{|}{Lys}}-AA_2-(Tyr)_s-(Cys)_t(COOH) \qquad (B)$$

wherein at least one of $AA_1$ and $AA_2$ is a sequence of from 1 to 12 amino acids corresponding to the peptide sequence adjacent to lysine 525 in human albumin, and if only one of $AA_1$ and $AA_2$ is such a sequence, then the other is a bond; wherein $q$ and $t$ are independently zero or 1; wherein $r$ and $s$ are, independently, zero, 1 or 2; wherein QNH represents the $\epsilon$-amino group in lysine and Q is hydrogen or 1-deoxyfructosyl; and wherein the N-terminal amino group in $(NH_2)AA_1$ and any lysine units in $AA_1$ or $AA_2$ can be glycosylated cr non-glycosylated. Preferably Q is the only glycosylation in the fragment. Normally, if one of $q$ and $t$ is 1 then the other is zero, and further when $q$ or $t$ is zero then $r$ or $s$ respectively, is also zero.

In a preferred embodiment, glycoalbumin is isolated from human blood and cleaved with an appropriate protease enzyme or enzymes to yield appropriately sized peptide fragments comprising glycosylated lysine 525 and adjacent amino acids. Because albumin is substantially resistent to proteolysis in its native conformation, it will normally be required to denature the protein to an extent necessary for the desired proteolysis to occur. The resulting glycopeptide fragments are isolated by conventional methods such as chromatography on gels having selective affinity for carbohydrate residues and high performance liquid chromatography (HPLC). Preparation of non-glycosylated peptide fragments by cleavage of non-glycosylated albumin with subsequent glycosylation of the fragments is also possible but obviously much less desirable because of the potential for glycosylation at sites in addition to lysine 525, e.g., other lysine units and the N-terminal amino group. The preferred glycosylated peptide fragments prepared by proteolysis of human albumin are:

$$(NH_2)\overset{QNH}{\overset{|}{Lys}}\text{-Gln-Thr-Ala-Leu-Val-Glu-Leu-Val-Lys}(COOH), \text{ and}$$

$$(NH_2)\text{Arg-Gln-Ile-Lys-}\overset{QNH}{\overset{|}{Lys}}\text{-Gln-Thr-Ala-Leu-Val-}$$
$$\text{Glu}(COOH),$$

wherein Q is 1-deoxyfructosyl. Chemical synthesis can also be used to prepare peptide fragments of desired sequence following conventional procedures and using commercially available peptide synthesis instrumentation. Following peptide synthesis, the resulting peptide is glycosylated under appropriate conditions, e.g., glucose-saturated pyridine, or glucose-saturated pyridine:acetic acid (1:1) for 48 hours at room temperature. During such *in vitro* glycosylation, the N-terminal amino group and the $\epsilon$-amino groups of all lysine units in the particular peptide including and in addition to the one corresponding to lysine 525 can be glycosylated. Such additional glycosylation can variously be tolerated during immunization by the animal responding non-specifically with respect to such glycosylation, due to the distal position of such glycosylation or its orientation in the peptide fragment, or can be selectively removed such as by protease cleavage of one or more terminal amino acids, particularly the potentially glycosylated N-terminal amino acid. The preferred glycosylated and non-glycosylated peptide fragments prepared by peptide synthesis are:

* (QNH)
$(NH_2)AA_3$-Gln-Ile-Lys-Lys-Gln-Thr-Ala-Leu-Val-
-Tyr-$AA_4$(COOH),

* (QNH)
$(NH_2)AA_4$-Glu-Arg-Gln-Ile-Lys-Lys-Gln-Thr-Ala-
-Leu(COOH),

* (QNH)
$(NH_2)$Gln-Ile-Lys-Lys-Gln-Thr-Ala-Leu-Val-Glu-
-Leu-$AA_4$(COOH),

* (QNH)
$(NH_2)AA_5$-Gln-Ile-Lys-Lys-Gln-Thr-Ala-Leu-Val-
-Glu(COOH),

(QNH)
$(NH_2)AA_6$-Lys-Gln-Thr-Ala-Leu-$AA_7$(COOH),
and (QNH)
*
$(NH_2)AA_8$-Arg-Gln-Ile-Lys-Lys-Gln-Thr,

wherein Q is hydrogen or 1-deoxyfructosyl; $AA_3$ is Lỹs-Glu-Arg, Arg, or a bond; $AA_4$ is Cys or a bond; $AA_5$ is Arg or a bond; $AA_6$ is Gln-Ile-Lỹs, Ile-Lỹs, Lỹs, or a bond; $AA_7$ is Tyr-Tyr-Cys, Tyr-Cys, Cys or a bond; $AA_8$ is Cys-Tyr-Tyr, Cys-Tyr, Cys, or a bond; and wherein the N-terminal amino group and any Lỹs units in the peptide are glycosylated or non-glycosylated. The most useful glycosylated peptides of the above formulae will have Q being 1-deoxyfructosyl and the N-terminal amino group and any other Lys units non-glycosylated.

The preferred non-glycosylated precursor peptides are of the formulae:

Lys-Glu-Arg-Gln-Ile-Lys-Lys-Gln-Thr-Ala-Leu-Val-Tyr-Cys, Cys-Glu-Arg-Gln-Ile-Lys-Lys-Gln-Thr-Ala-Leu, Gln-Ile-Lys-Lys-Gln-Thr-Ala-Leu-Val-Glu-Leu-Cys, and Lys-Gln-Thr-Ala-Tyr-Tyr-Cys, Lys-Lys-Gln-Thr-Ala-Tyr-Tyr-Cys, Ile-Lys-Lys-Gln-Thr-Ala-Tyr-Tyr-Cys, Gln-Ile-Lys-Lys-Gln-Thr-Ala-Tyr-Tyr-Cys, and Cys-Tyr-Tyr-Arg-Gln-Ile-Lys-Lys-Gln-Thr.

A more specific glycosylation of the lysine corresponding to lysine 525 is obtained by combinations of solution and solid phase peptide synthesis coupled with α-amino blocked, ε-amino 1-deoxyfructosyl lysine. In this synthesis, $AA_2$-$(Tyr)_r$- $(Cys)_s$-(COOH) is prepared by conventional synthese Lysine (α-amino blocked ε-deoxyfructosyl lysine) is prepared separately and is coupled using classical solution phase chemistry to the amino terminus of such peptide producing

$$\begin{array}{c} \text{QNH} \\ | \\ \text{(B)-Lys-AA}_2\text{-(Tyr)}_s\text{-(Cys)}_t\text{-(COOH)} \end{array}$$

where B is a blocking group on the α-amino group of lysine, such as t-butyloxycarbonyl (t-BOC); dinitrophenyl (DNP); p-fluorenylmethoxycarbonyl (fMOC); or other suitable blocking groups which can be removed without altering the QHN-Lys (1-deoxyfructosyl lysine). The blocking group can be removed by selected chemistries (based on the particular blocking group) that do not alter the 1-deoxyfructosyl structure of lysine. This peptide can be used directly as an immunogen or in an immunoassay or can be extended by the addition of $NH_2$-$(Cys)_q$-$(Tyr)_r$-$AA_1$ to produce

$$\begin{array}{c} \text{QHN} \\ \text{NH}_2\text{-(Cys)}_q\text{-(Tyr)}_r\text{-AA}_1\text{-Lys-AA}_2\text{-(Tyr)}_s\text{-(Cys)}_t\text{-(COOH)} \end{array}$$

The addition can be by a sequential extension using classical solution or solid phase peptide synthesis or by a segment condensation where preformed $NH_2$-$(Cys)_q$-$(Tyr)_r$-$AA_1$ is condensed onto QHN-Lys-$AA_2$-$(Tyr)_s$-$(Cys)_t$-(COOH) to yield the final product.

The introduction of terminal Cys units enables the selective coupling of the peptide to immunogenic carrier materials, such as through bifunctional linking agents well known in the art, e.g., m-maleimidobenzoyl-N-sulfosuccinimide ester(MBS). Alternatively, the peptide fragments can be coupled through the C-terminal carboxyl group using conventional peptide condensation methods, e.g., carbodiimide coupling reagents. Other linking methods conventionally known can also be used.

It will also be generally preferred to introduce one, two, or more Tyr units either as terminal units on the peptide or adjacent to the albumin-specific sequence and/or the terminal amino acid used for the coupling of the peptide to immunogenic carrier materials, e.g., adjacent to a terminal Cys unit. The presence of Tyr units in the non-specific region of the peptide residue is believed to enhance the immunogenicity of the glycosylated specific region of the peptide thereby stimulating the antibody response.

Therefore, in particularly preferred embodiments $AA_1$ and $AA_2$ in the formulas herein will, respectively, begin with the sequence Cys-$(Tyr)_r$- and end with the sequence -$(Tyr)_s$-Cys, where $r$ and $s$ are integers from 1 to as many as 10 or more, preferably 1 or 2.

The immunogen used to stimulate production of appropriate immunoglobulins in the most general sense will comprise one or more of the glycosylated peptide residues chemically linked to an immunogenic carrier material. The general formula for such immunogen is:

$$\text{Carrier}_m \left[ \text{R}_m\text{—AA}_1\text{-}\underset{}{\overset{\text{Glyco-(NH)}}{\text{Lys}}}\text{-AA}_2\text{—R}_n \right]_p \text{Carrier}_n \qquad \text{(C)}$$

wherein Glyco-(NH), $AA_1$ and $AA_2$ are as defined previously provided that $AA_1$ and $AA_2$ can be terminal amino or carboxyl groups when $m$ or $n$ is zero, respectively; R is a bond or linking group; Carrier is an immunogenic carrier material; one of m and n is 1 and the other is zero; and $p$ is on the average from 1 to the number of available coupling sites on Carrier. The residues $AA_1$ and $AA_2$ can include Tyr and Cys units as discussed above.

The immunogenic carrier material can be selected from any of those conventionally known having functional groups available for coupling to the glycosylated peptide residue. In most cases, the carrier will be a protein or polypeptide, although other materials such as carbohydrates, polysaccharides, lipopolysaccharides, nucleic acids, and the like of sufficient size and immunogenicity can likewise be used. For the most part, immunogenic proteins and polypeptides will have molecular weights between 4,000 and 10,000,000, preferably greater than 15,000, and more usually greater than 50,000. Generally, proteins taken

from one animal species will be immunogenic when introduced into the blood stream of another species. Particularly useful proteins are albumins, globulins, enzymes, hemocyanins, glutelins, proteins, having significant nonproteinaceous constituents, and the like. Further reference for the state-of-the-art concerning conventional immunogenic carrier materials and techniques for coupling haptens thereto may be had to the following: Parker, Radioimmunoassay of Biologically Active Compounds, Prentice-Hall (Englewood Cliffs, New Jersey USA, 1976); Butler, J. Immunol. Meth. 7:1-24(1976); Weinryb and Shroff, Drug Metab. Rev. 10:271-283(1974); Broughton and Strong, Clin. Chem. 22:726-732(1976); and Playfair et al, Br. Med. Bull. 30:24-31(1974).

The letter p in formula (C) represents the number of glycosylated residues that are conjugated to the carrier, i.e., the epitopic density of the immunogen, and will range from 1 to the number of available coupling sites on the carrier and can be as high as 5000 in the case of certain high molecular weight synthetic polypeptides such as polylysine. The epitopic density on a particular carrier will depend upon the molecular weight of the carrier and the density of available coupling sites. Optimal epitopic densities, considering the ease and reproducibility of synthesis of the immunogen and antibody response, fall between about 10% and about 50% of the available coupling groups on the carrier involved.

Linking group R can be essentially any convenient and stable structure. Such linking group R will usually be in the form of a bond or an aliphatic chain comprising between 1 and about 20 atoms, excluding hydrogen, and including heteroatoms such as nitrogen, oxygen, and sulfur. The glycosylated residue can be joined through a variety of groups to form linking chain R, including methylene, ether, thioether, imino, and the like. One skilled in the art will have a wide variety of linking groups from which to choose to prepare the immunogen. Normally, the glycosylated peptide will be prepared terminating in a functional group such as amino, carboxyl, thiol, hydroxyl, or maleimido which is active in a coupling reaction to an appropriate group in the carrier molecule.

Particularly preferred immunogens of formula (C) are those wherein:

(a) $AA_1$ is a terminal amino group, $AA_2$ is Gln-Thr-Ala-Leu-Val-Glu-Leu-Val-Cys, m is zero, and n is 1;

(b) $AA_1$ is ($NH_2$)Arg-Gln-Ile-Lys, $AA_2$ is Gln-Thr-Ala-Leu-Val-Glu, m is zero, and n is 1;

(c) $AA_1$ is ($NH_2$)L*ys-Glu-Arg-Gln-Ile-L*ys, $AA_2$ is Gln-Thr-Ala-Leu-Val-Tyr-Cys, m is zero, and n is 1;

(d) $AA_1$ is Cys-Glu-Arg-Gln-Ile-L*ys and $AA_2$ is Gln-Thr-Ala-Leu(COOH), m is 1, and n is zero; wherein L*ys is a glycosylated or non-glycosylated lysine unit;

(e) $AA_1$ is Gln-Ile-Lys, Ile-Lys, Lys, or a terminal amino group, $AA_2$ is Gln-Thr-Ala-Leu-Tyr-Tyr-Cys, m is zero, and n is 1;

(f) $AA_1$ is Cys-Tyr-Tyr-Arg-Gln-Ile-Lys, $AA_2$ is Gln-Thr, m is 1, and n is zero.

The antibody selected for use in an immunoassay can be of any immunoglobulin class, e.g., IgG, IgM, and so forth, and of any subclass thereof. Normally, the antibody will be of the IgG class and if desirable any fragment of such antibody can be used which contains an antibody combining site, e.g., Fab, F(ab'), and $F(ab')_2$. The selected antibody reagent can be used in any immunoassay method for the purpose of determining glycoalbumin in a biological fluid. Such immunoassay methods include the more classical techniques such as immunodiffusion, immunoelectrophoresis, agglutination techniques, and complement fixation, as well as more current techniques involving the use of specifically detectable labels such as radioimmunoassay and nonradioisotopic methods. The latter techniques can be practiced in a wide variety of formats such as the competitive binding format in which a labeled reagent is made to compete with the glycosylated analyte for binding to the antibody reagent. The amount of labeled reagent bound to the antibody reagent, or the free-species, consisting of the labeled reagent which is not so bound, is measured appropriately and can be functionally related to the amount of glycosylated analyte in the sample.

In radioimmunoassays, the free-species and bound-species must be physically distinguished or separated in order to measure the label since the signal generated by the label is qualitatively the same in both species. Such a technique is known in the art as heterogeneous because of the phase separation requirement. Other heterogeneous immunoassay techniques are known including enzyme-labeled immunoassays, sometimes referred to as ELISA techniques (see U.S. Pat. No. 3,654,090), and fluorescent immunoassays (see U.S. Pat. Nos. 4,201,763; 4,133,639 and 3,992,631), particularly particle concentration fluorescent immunoassays (see published European Patent Application 124,050).

Fairly recently, numerous immunoassay techniques have been developed which obviate the separation step through the use of a label whose detectable signal is modulated upon binding of the labeled reagent by a binding partner, e.g., antibody. Such techniques have become known as homogeneous and are advantageous when used in the present invention because separations are not required and radioisotopes are not involved. Some such techniques are fluorescence quenching and enhancement (see U.S. Pat. No. 4,160,016), energy transfer immunoassay (see U.S. Pat. No. 3,996,345), and double antibody steric hindrance immunoassay (see U.S. Pat. Nos, 3,935,074 and 3,998,943). Particularly preferred homogeneous

immunoassay techniques are those employing a label which is a participant in an enzyme-catalyzed reaction. Examples are the substrate-labeled immunoassay (see U.S. Pat. No. 4,279,992 and U.K. Patent Spec. 1,552,607), the prosthetic group (FAD)-labeled immunoassay (see U.S. Pat. No. 4,348,565), the enzyme modulator-labeled immunoassay, e.g., using inhibitor labels (see U.S. Pat. Nos. 4,134,972 and 4,273,866), and enzyme-labeled immunoassay (see U.S. Pat. No. 3,817,837).

The monoclonal antibodies of the present invention are specific for binding to the glycosylated peptide residue comprising lysine 525 in human albumin. It may be required in some cases, or may be particularly desirable to improve assay performance, to expose the glycosylated lysine 525 epitope in the native albumin molecule in order to perform the desired immunoassay. Steric access to the epitope can be obtained in any effective manner. Exposure of the epitope in the intact protein is understood to be accomplished by a physical or chemical denaturation or digestion at least in the region of the epitope. Such denaturation or digestion can be localized to the region of the epitope or can involve a more general, or even substantially complete denaturation of the tertiary, and additionally the secondary, structure of the protein, or partial or complete digestion of the protein.

When necessary or desirable, denaturation can be accomplished in a variety of ways including conventional treatment of the protein by physical means such as heat, sonication, high or low pH and, as is preferable, chemical denaturation by interaction with a chaotropic agent or chaotrope in solution. Useful chaotropic agents will normally include, without limitation, guanidine, urea, and various detergents such as sodium dodecylsulfate (SDS) and others, without limitation, including deoxycholate and certain bile salts, 3-(3-cholamidopropyl)-dimethylammonio-1-propanesulfonate, organic solvents such as methanol, propanol, acetonitrile and certain salts such as potassium thiocyanate. Non-ionic detergents such as Triton X-100, nonidet NP-40 and octyl-glucosides can also function as protein denaturants. Inclusion of reagents (e.g., mercaptoethanol or dithiothreitol) that reduce disulfide bonds can be effective promoters of the denaturation process. Protein denaturation can usually be most effectively accomplished if combinations of chemical and/or chemical and physical means are used (e.g., guanidine and heat, guanidine and SDS, or guanidine and dithiothreitol). Of course, denaturing conditions which result in substantial insolubilization, aggregation, or precipitation of the protein such that an insignificant amount of the exposed epitope is accessible to the solution for antibody binding will be avoided. A sufficient amount of the denatured protein must remain in solution or suspension in order to obtain useful immunobinding. The extent of solubilization necessary will depend upon the circumstances of the intended or desired binding.

The present invention will now be illustrated, but is not intended to be limited, by the following examples.

## EXAMPLES

### EXAMPLE 1: Isolation of naturally occurring albumin and glycoalbumin

Whole blood from a normal human donor was collected into a citrate-dextrose solution and separated into red cells and plasma by centrifugation. The plasma fraction was chromatographed on a 100 ml boronate-agarose column (Glycogel, Pierce Chemical Co., Rockford, IL, USA) in 0.25M ammonium acetate, 50mM $MgCl_2$, pH 8.0. The bound fraction (containing primarily glycoalbumin and immunoglobulins) was eluted with 0.1M Tris, 0.2M sorbitol, 10mM EDTA, pH 8.0. To separate glycoalbumin from immunoglobulins, the bound fraction was dialyzed into 50mM sodium phosphate, pH 8.0, and loaded onto a 100 ml DEAE Affi-gel blue column (Bio-Rad Laboratories, Richmond, CA USA). The column was eluted with 50mM sodium phosphate buffer, pH 8.0, containing a gradient of zero to 1.4M NaCl. The eluate was monitored at 280nm and the glycoalbumin identified by SDS-polyacrylamide gel electrophoresis. In some experiments the plasma was chromatographed on DEAE Affi-gel blue before the Glycogel column. The order of separation does not effect the final purity of the albumin or glycoalbumin.

Non-glycosylated albumin was purified from the Glycogel non-bound fraction subsequently separated on a DEAE-affi-gel blue column as above. The albumin and glycoalbumin were dialyzed into phosphate buffered saline (PBS, 7.2mM $Na_2HPO_4$, 2.8mM $NaH_2PO_4$, 127mM NaCl, pH 7.4) containing 0.05% sodium azide, lyophilyzed and stored at -80 °C until further use.

### EXAMPLE 2: Generation and isolation of enzymatically cleaved glycopeptides from glycoalbumin

Human serum albumin has 59 lysine residues, one of which is the primary site of glycosylation in the native molecule. To produce a peptide containing the glycosylated lysine (at position 525) we have used the known protein sequence of albumin coupled with the unique specificity of the two proteases trypsin and

Staphylococcus aureus V8 protease. Trypsin cleaves the peptide bond on the carboxy-side of lysine and arginine, but does not cleave on the carboxy-side of a glycosylated lysine. Staph V8 cleaves on the carboxy-side of aspartic acid and glutamic acid. Therefore a tryptic digest or a V8 digest should produce the respective peptides containing the glycosylated lysine at position 525 [see Structures (1) and (2) in the drawing - the solid and open triangles indicate the cleavage sites for trypsin and V8 protease, respectively].

In its native conformation albumin is substantially resistent to proteolysis. To optimally expose the protein to the proteases glycoalbumin was denatured in 8M urea, 5mM dithiothreitol (DTT), 0.1M ammonium bicarbonate, pH 7.85, for 2 hours at ambient temperature. The denatured protein solution is slowly added to 0.1M ammonium bicarbonate, pH 7.85, containing 1:50 ratio (weight of enzyme: weight of glycoalbumin) of protease to protein. The resulting final concentration of urea is 0.8M and the solution is maintained at 37°C for 16 hours. Enzyme (equivalent in weight to the first addition) is then added and the solution incubated for 8 hours. The solution is applied to a boronate Affi-gel 601 column (BioRad, Richmond, CA, USA) to selectively bind the carbohydrate containing peptides. The column is thoroughly washed in 50mM ammonium bicarbonate and the bound peptides eluted with 0.1M acetic acid. The eluted peptides were dried, resuspended in 20mM potassium phosphate, pH 7.0, and injected onto an Altex-ODS (4.1mm × 25cm) HPLC column (Rainin, Emeryville, CA, USA). A gradient of 1% acetonitrile/minute to a final concentration of 60% acetonitrile in the above buffer was used to elute the bound components. Fractions were collected, dried, and hydrolyzed under argon for 24 hours in 6N HCl containing 0.02% phenol. The hydrolysates were dried and analyzed using an OPA precolumn derivitization procedure (Benson and Hare, Proc. Natl. Acad. Sci. 72:619-622, 1975) and separation of the amino acid - OPA adducts on HPLC C-18 column (Supelco, Bellefonte, PA, USA). Amino acids were identified and quantitated by comparison to known standards (Standard H; Pierce Chemical Co., Rockford, IL, USA). The expected and found values for the tryptic and V8 peptides are shown in Table 1.

## Table 1

### STAPH V8 PROTEASE PEPTIDE

| AMINO ACID | EXPECTED | FOUND |
|---|---|---|
| GLU | 3 | 3.1 |
| THR | 1 | 1.0 |
| ARG | 1 | 1.2 |
| ALA | 1 | 1.0 |
| VAL | 1 | 1.0 |
| ILE | 1 | 0.7 |
| LEU | 1 | 1.0 |
| LYS | 2 | 1.9 |

### TRYPTIC PEPTIDE

| AMINO ACID | EXPECTED | FOUND |
|---|---|---|
| GLU | 2 | 2.1 |
| THR | 1 | 1.0 |
| ALA | 1 | 1.2 |
| VAL | 2 | 1.8 |
| LEU | 2 | 2.0 |
| LYS | 2 | 2.0 |

EXAMPLE 3: Coupling of proteolytically cleaved glycopeptides to carrier protein

The C-terminal carboxylic acid was selectively activated and coupled to the amines of carrier proteins (Cell 34:587-596, 1983). Two mgs of EDCI (1-ethyl-3-(3-dimethylaminopropyl)-carbodiimide, Pierce Chemical Co.) in 200µl of 0.01N HCl was added to 2mg of the dry glycopeptide of Example 1. This solution was quickly added to 6mg keyhole limpet hemocyanin (KLH) in 2mL $H_2O$ resulting in a precipitate. The pH of the solution was raised to 9.0 with 0.1M ammonium carbonate, stirred for 3 hours and then dialyzed against PBS. The thiobarbituric acid test (Fluckinger and Winterhalter, FEBS Letters 71:356-360, 1976) indicated at least 2 carbohydrates/100,000MW of KLH using fructose as the standard.

EXAMPLE 4: Chemical synthesis of albumin peptides

(a) Peptides were synthesized on the Applied Biosystems 430A Peptide Synthesizer (Applied Biosystems, Foster City, CA, USA).

The carboxyl terminus amino acid was coupled to the resin by a phenylacetamidomethyl linkage with a substitution of 0.7 mmoles per gram resin. Typically 0.5 mmol of peptide was produced per synthesis. The N-terminal t-BOC was removed with 60% trifluoroacetic acid (TFA) in dichloromethane (DCM) and the $\alpha$-amine neutralized with 10% diisopropylethylamine in dimethylformamide (DMF). t-BOC amino acids (2 mmol) were converted to preformed symmetrical anhydrides by the addition of 1 mmole dicyclohexylcarbodiimide in 2 ml dichloromethane. The side chains of the t-BOC amino acids were protected as follows: Arg(TOS), Asp(OBzl), Cys(4-CH Bzl), Glu(OBzl), His(TOS), Lys(Cl-Z), Ser(Bzl), Thr(Bzl), and Tyr(Br-Z). The t-BOC amino acids Ala, Asn, Gln, Gly, Ile, Leu, Met, Phe, Pro, Trp, and Val were not protected. The dicyclohexylamine salt of t-BOC-L-His(Tos) was converted to the free acid by ion-exchange on AG-50-X8-(H+) resin (Bio-Rad) within one hour of coupling. The t-BOC amino acid Asn, Arg, and Gln (2 mmol) were coupled using preformed hydroxybenztriazole (HOBt) active esters formed by the addition of 2 mmol HOBt and 2 mmol DCC. The N-terminal t-BOC was removed from the completed peptide and the peptide resin dried overnight in vacuo.

The peptides were fully deprotected and cleaved from the resin by treatment with anhydrous HF containing 10% anisole for 60 minutes at 0 °C. The resin was washed with ethyl acetate and the peptide extracted from the resin with 1.0N acetic acid. The extract was immediately frozen in liquid nitrogen, lyophilized, and stored at -20 °C until further use.

(b) ALB K14C

LYS-GLU-ARG-GLN-ILE-LYS-LYS-GLN-THR-ALA-LEU-VAL-TYR-CYS

This 14 amino acid peptide has a 12 amino acid albumin sequence with a penultimate TYR and a C-terminal CYS. The sulfhydryl of the CYS can be selectively coupled to carriers or to fluorescent reagents (see Examples 6 and 9). During in vitro glycosylation the N-terminal and ε-amino groups of the N-terminal lysine are likely to be glycosylated [see structure (3) in the drawing]. However, proteolytic digestion with V8 will remove the terminal LYS-GLU or digestion with trypsin will remove the terminal LYS-GLU-ARG resulting in a shorter peptide which lacks the potentially glycosylated N-terminal lysine.

(c) ALB C11L

CYS-GLU-ARG-GLN-ILE-LYS-LYS-GLN-THR-ALA-Leu

This peptide has 10 amino acids of the albumin sequence plus an N-terminal CYS for selective coupling. The N-terminal amino group of CYS is likely to be glycosylated but since it is proximal to the carrier or label it should not have an effect on the production of antibodies specific for glycoalbumin [see structure (4) in the drawing]. This peptide lacks the C-terminal hydrophobic amino acids VAL and TYR and has better solubility in aqueous and pyridine buffers.

(d) ALB Q12C

GLN-ILE-LYS-LYS-GLN-THR-ALA-LEU-VAL-GLU-LEU-CYS

This peptide has 11 amino acids of the albumin sequence plus a C-terminal CYS for selective coupling [see structure (5) in the drawing]. The desired lysine epitope (525) has been placed 4 amino acids from the N-terminus increasing its exposure and its antigenicity.

(e) ALB R11E

ARG-GLN-ILE-LYS-LYS-GLN-THR-ALA-LEU-VAL-GLU

This peptide has 11 amino acids of the albumin sequence [see structure (6) in the drawing]. The N-terminal ARG can be removed using trypsin if the N-terminal amino group becomes glycosylated during in vitro glycosylation. The desired lysine epitope (525) will be 4 amino acids from the N-terminus increasing its exposure and antigenicity. This peptide also has good solubility in aqueous and pyridine buffers.

(f) ALB Q9C

L*YS-GLN-THR-ALA-LEU-VAL-GLU-LEU-VAL-CYS

N-t-BOC-L-Lysine (Sigma Chemical Co., St. Louis, MO, USA) was incubated in 95% pyridine, 5% acetic acid or 10% pyridine in absolute methanol saturated with glucose for 7 days at 50 °C. The solution was dried to a syrup and N-t-BOC-ε-1-deoxyfructosyl lysine purified by HPLC on a C-18 (Altex ODS, 4.1mm ×

25cm) column (Solvent A = 50mM triethylamine-acetate pH 6.0; solvent B = A:acetonitrile 50:50). The product has an Rf of 0.81 silica gel using chloroform:methanol:acetic acid (14:5:1) and was not reactive to amine detecting reagents unless briefly exposed to HCl vapors and heating to 100 °C.

The N-t-BOC-ϵ-1-deoxyfructosyl lysine is coupled to the N-terminus of the synthesized peptide GLN-THR-ALA-LEU-VAL-GLU-LEU-VAL-CYS. This peptide has eight amino acids of the albumin sequence plus a C-terminal CYS for coupling [see structure (7) in the drawing]. Following removal of t-BOC the resulting peptide has lysine 525 glycosylated solely on the ϵ-amino group. For coupling, 1 equivalent of N-t-BOC-ϵ-deoxyfructosyl lysine in dichloromethane is reacted with 0.5 equivalent dicyclohexylcarbodiimide for 15 minutes at room temperature under argon. An equal volume of dimethyl formamide is added followed by 0.25 equivalent moles of the synthesized peptide. After 30 minutes the solution is dried, resuspended in 25% TFA in dichloromethane for 30 minutes, dried again and the product purified on HPLC on a C-28 column.

(g) Tryptic condensation of the glycosylated product of ALB Q9C ($AA_1$) with $AA_2$.

The N-terminal extension peptide B-GLN-ILE-LYS is synthesized by conventional solution or solid phase peptide synthesis. The HPLC purified peptide is incubated with TPCK-treated trypsin (Cooper Biomedical, Malvern, PA, USA) in 30% isopropanol or other suitable organic solvent (Fruton, Advances in Enzymology 53:239-306, 1981) and equal molar amounts of LYS-GLN-THR-ALA-LEU-GLU-LEU-VAL-CYS (the product of example 4f) are added. After twenty four hours the resulting product B-GLN-ILE-LYS-LYS-GLN-THR-ALA-LEU-VAL-GLU-LEU-VAL-CYS is isolated by HPLC. The terminal blocking group (B) can be removed by procedures that do not affect the 1-deoxyfructosyl residue on lysine 525.

(h) ALB Q7C

LY̌S-GLN-THR-ALA-LEU-TYR-TYR-CYS

This peptide is used in experiments analogous to those described for (f) ALB Q9C where N-t-BOC-ϵ-1-deoxyfructosyl lysine is coupled to the N-terminal GLN using dicyclohexylcarbodiimide as described in (f). This peptide (ALB Q7C) has at the C-terminus a TYR-TYR-CYS structure which is thought to potentiate the immune response against a synthetic peptide immunogen. The small size of the albumin part of the sequence (5 amino acids) should provide an epitope of restricted size and thereby focus the immune response to the glycosylated lysine residue.

(i) ALB K8C

LYS-GLN-THR-ALA-LEU-TYR-TYR-CYS

ALB K8C is a small peptide-containing the desired glycosylated lysine on the N-terminus and the non-albumin TYR-TYR-CYS sequence at the C-terminus. This peptide is highly soluble in 0.1% TFA and absolute methanol which allow it to be purified by HPLC and glycosylated in the respective solvents.

(j) ALB K9C

LYS-LYS-GLN-THR-ALA-LEU-TYR-TYR-CYS

ALB K9C has the properties of ALB K8C plus an additional lysine residue at the N-terminus.

(k) ALB I10C

ILE-LYS-LYS-GLN-THR-ALA-LEU-TYR-TYR-CYS

ALB I10C has the properties of (j) ALB K9C but has an additional ILE residue at the N-terminus.

(l) ALB Q11C

GLN-ILE-LYS-LYS-GLN-THR-ALA-LEU-TYR-TYR-CYS

ALB Q11C has the properties of (k) I10C but has an additional GLN residue at the N-terminus.

(m) ALB C10T

CYS-TYR-TYR-ARG-GLN-ILE-LYS-LYS-GLN-THR

ALB C10T was synthesized with the non-albumin sequence (CYS-TYR-TYR) on the N-terminus to favor antibodies that may bind preferentially to the glycosylated lysine and the C-terminal sequence of this peptide.

EXAMPLE 5: Glycosylation of synthetic peptides

The four synthetic peptides listed in Table 2 were glycosylated as indicated. The Q9C peptide was prepared by glycosylation as described in Example 4(f).

**TABLE 2**

| | | K14C | C11L | Q12C | R11E |
|---|---|---|---|---|---|
| (a) | pyridine, 0.25M glucose | + | + | + | + |
| (b) | 50% pyridine, 50% $H_2O$, 0.125M glucose | + | | + | + |

**TABLE 2 (cont'd)**

| | | K14C | C11L | Q12C | R11E |
|---|---|---|---|---|---|
| (c) | PBS, 1.0M glucose | + | + | + | + |
| (d) | 95% pyridine, 5% acetic acid 0.25M glucose, pH 7.0 | + | + | + | + |

Reactions were from ambient to 50°C and from 1-20 days. Samples were dried to a syrup and injected onto an Altex C-18 (1 × 25cm) using a 0.1% TFA to 0.1% TFA, 60% acetonitrile gradient. Peak fractions were collected, analyzed for carbohydrate and used in the production of glycopeptide-MBS-carrier protein immunogens.

The non-glycosylated peptides were also coupled to KLH-MBS and subsequently glycosylated in vitro in PBS containing 1.0M glucose (pH 9.5 or 7.4) at 37°C for 7-14 days. Thiobarbituric acid analysis indicated 10-40 carbohydrates/100,000MW KLH.

Peptides of Example 4 (i) through (m) were glycosylated at elevated temperatures (50-80°C) 70°C in glucose saturated methanol for 24 hours. The methanol was removed by negative pressure and the glycopeptide purified by HPLC. This has been shown to be highly effective in attaching glucose to the lysine amino groups in synthetic peptides and to the N-t-BOC-L-LYSINE of Example 4 (f).

Glycosylation of N-t-BOC-L-LYSINE in glucose saturated methanol 50-80°C for 24 hours was especially effective. Sequence analysis (Example 6 below) identified the product (after removal of the α-amine

protecting group) as ε-deoxyfructosyl lysine. Fast atom bombardment mass spectrascopy also gave the predicted molecular weight of the glycoslyated N-t-BOC-L-LYSINE derivative.

EXAMPLE 6: Sequence analysis for the determination of the position and quantitation of glycosylated lysines

A method was developed for the determination of the position and the quantitation of glycosylated lysine residues using automated gas-phase Edman degradation sequencing procedures. During conventional sequence analysis both amino groups on lysine are reactive with PITC (phenylisothiocyanate) forming a lysine with a PTC group (phenylthiocarbamyl) on the ε-amino group and a PTH (phenylthiohydantoin) on the α-amino group. A glycosylated lysine, however, will not have the PTC group on the ε-amino because the carbohydrate blocks that amine from reacting with PITC.

The lysine product is therefore PTH-lysine. In the sequence analysis of the naturally occuring glycoalbumin peptides of Example 2 applicants have identified the PTH-lysine residue since it has a unique chromatographic retention time on the C-18 reverse phase column used to separate and quantitate the various PTH-amino acids. All glycosylated synthetic peptides were sequenced to identify the particular glycosylated lysine in multi-lysine peptides and to quantitate the ratio of lysine to glycolysine. The results indicate that greater than 75% of the lysines have the correct glycosylation reaction product on lysine when the glycosylation is done in methanol.

EXAMPLE 7: Coupling of synthetic glycopeptides to carrier proteins

Synthetic glycopeptides containing CYS are coupled to carriers as described by Lerner et al (Proc. Natl. Acad. Sci. 78: 3403, 1981). Briefly KLH is reacted with a 50-fold molar excess of sulfo-MBS (Pierce Chemical Co.) for 25 minutes at ambient temperature in 50mM sodium phosphate, pH 6.2, and the KLH-MBS conjugate separated from unreacted sulfo-MBS by gel filtration in the same buffer. The KLH-MBS conjugate was immediately added to the dried glycopeptide (2-fold molar excess of glycopeptide to maleimide of carrier) and reacted overnight at room temperature.

For peptides lacking CYS, the C-terminal carboxylic acid was coupled to the amino groups of carrier proteins as described in Example 3.

EXAMPLE 8: Immunization

The selected glycopeptide MBS-KLH conjugate of Example 7 was emulsified with an equal volume of Freund's complete adjuvant. Mice (BALB/cBy) were injected with 200µg of conjugate and were boosted at 30 and 60 days with conjugate in incomplete adjuvant. Three days prior to fusion mice were injected with 50µg IV. The mice were sacrificed and their spleens used for fusions according to Kohler and Milstein, Nature 256: 495 (1975).

EXAMPLE 9: Screening of hybridoma supernatants for antibodies specific for glyco-albumin

(a) ELISA assay

Albumin and glycoalbumin of Example 1 were coated onto separate polystyrene microtiter plates (2ug per 100 microliters/well) overnight at 4°C. Plates were washed with PBS, 0.05% Tween-20. Supernatants from each cell line were incubated in albumin or glycoalbumin coated plates for 60 minutes. The plates were washed 4 times with PBS + 0.05 Tween-20 and 200 microliters of secondary antibody added to each well (1:2000 dilution of rabbit anti-mouse IgG-peroxidase, Miles Laboratories, Inc., Elkhart, IN, USA). After 60 minutes the plates were washed 4 times in PBS + 0.05% Tween and 200 microliters of substrate solution (24.3mM citric acid, 51.4mM sodium phosphate, pH 5.3, containing 2.2mM o-phenylenediamine and 5.2mM hydrogen peroxide) was added. The reaction was terminated after 20 minutes by adding 50 microliters of 8M $H_2SO_4$ and the product of the peroxidase reaction read at 492nm. The monoclonal antibodies that are specific for glycoalbumin react with glycoalbumin and not albumin.

(b) Particle concentration fluorescent immunoassay

Albumin and glycoalbumin were coated onto separate polystyrene particles (Pandex Laboratories, Mundelein, IL, USA). Hybridoma supernates (20 microliters) were added to each well followed by 20

microliters of albumin or glycoalbumin coated particles. After 30 minutes a 1:5000 dilution of goat anti-mouse IgG-FITC (see Example 10) was added and the incubation continued for an additional 30 minutes. All the non-bound reactants were removed by filtration and the fluorescence measured. In a specific response, antibodies in the hybridoma supernate bind to glycoalbumin but not albumin coated particles.

(c) Particle concentration fluorescent immunoassay with prior dissociation of albumin/glycoalbumin from monoclonal antibody

Goat anti-mouse particles (Pandex Laboratories) are incubated with hybridoma supernates to capture the mouse antibodies. A percentage of mouse monoclonal antibodies are bound to the naturally occurring glycoalbumin in the cell culture media used to grow the hybridoma cells. Non-bound components are separated by filtration leaving the goat anti-mouse particles, with bound mouse immunoglobulins from the hybridoma media and in turn the glycoalbumin bound to the mouse immunoglobulin. Twenty (20) microliters of 100mM glycine, pH 3.0, is added to dissociate all complexes. Twenty minutes later, 20 microliters of 50mM Tris base containing glycoalbumin labeled with fluorescein using the sulphydryl specific fluorescein-5-maleimide is added. The resulting pH of 7.5 renatures the mouse immunoglobulin which preferentially binds to the excess of fluorescnet glycoalbumin. The mouse immunoglobulin-fluorescent glycoalbumin complex is captured by the existing goat anti-mouse particles or by adding fresh goat anti-mouse Ig particles. The non-bound reagents are removed by filtration and the signal is proportional to the mouse anti-glycoalbumin antibodies present in the hybridoma supernate.

(d) Improved growth medium

Fetal calf serum (20%) used to maintain and grow myeloma cells and hybridoma cells has a significant concentration of bovine albumin and presumably glycoalbumin which is known to have the same amino acid sequence as human albumin surrounding thing glycosylated lysine at position 525. It is therefore highly probable that the small amount of anti-glycoalbumin antibodies secreted into the tissue culture media immediately bind to the glycosylated albumin molecules and cannot be detected in the standard ELISA assay.

To eliminate the binding of antibody to media glycoalbumin, myeloma cells and hybridoma cells were adapted to growth in serum (and albumin) free media. The media currently being used is commercially available (HL-1, Ventrex, Portland, Maine USA). The screening of hybridoma supernates in the HL-1 media simplifies the identification of clones secreting anti-glycoalbumin antibodies.

(e) Growth of hybridoma cells in glycoalbumin containing media

As discussed in Example 9 (d) the presence of glycoalbumin in the media may prevent the detection of anti-glycoalbumin specific antibodies. It is therefore necessary to remove the glycoalbumin from the media by a selection adsorption process. This is accomplished by selectively absorbing the albumin and glycoalbumin from fetal calf serum by passage down an Affi-Gel Blue column (BioRad Labs, Richmond, CA, U.S.A.) using the manufacturer's directions. The albumin fraction has an affinity for the reactive blue dye under these conditions but can be eluted using 1.4 M sodium chloride. The eluted fraction containing approximately 90% albumin and 10% glycoalbumin is applied to a Glyco-Gel B (boronate column - Pierce Chemical Co., Rockford, IL, U.S.A.). This column selectively binds glycoalbumin. The non-bound fraction contains nonglycoalbumin and is added back to the Affi-Gel non-bound fraction which contains all serum components except albumin. The final mixture is fetal calf serum deleted of glycoalbumin and is used to prepare the media for the growth of hybridomas producing anti-glycoalbumin specific antibodies.

EXAMPLE 10: Fluorescent labeling of synthetic glycopeptides

The glycopeptides can be conveniently labeled using sulfhydryl specific fluorescein conjugates. A two fold molar excess of fluorescein-5-malemide in dimethylformamide (40mg/ml) is added to glycopeptide (10mg/ml) in 100mM sodium phosphate, 5mM EDTA, pH 7.1. The sample is incubated for 20 hours at room temperature. The glycopeptide-fluorescein conjugate is purified by HPLC on an Altex C-18 4.1mm × 25cm column using 20mM sodium phosphate to 20mM sodium phosphate, 50% acetonitrile gradient.

EXAMPLE 11: Immunoassay for glycoalbumin in clinical specimens

Antibody specific for glycoalbumin is coated onto polystyrene particles (0.8um) Pandex Laboratories). Antibody coated beads (20 microliters) are incubated with an appropriately diluted blood specimen, e.g., 1:800. The specimen may be serum, plasma or whole blood. The diluent may be in physiological buffers or in denaturing solutions that lyse red blood cells and optimally expose the glycoalbumin epitope. Following an appropriate incubation (e.g., 5 minutes), synthetic glycopeptide fluorescein conjugate (Example 10) is added to bind to unoccupied antibody binding sites on the particles. After a further incubation (e.g., 20 minutes), all non-bound reactants are removed by filtration and the fluorescein quantitated. The fluorescein signal is therefore inversely proportional to the amount of competing glycoalbumin in the clinical specimen.

It will be understood that the specification and examples are illustrative, but not limiting with regard to the present invention and that other embodiments and modifications within the spirit and scope of the present invention will be evident to those skilled in the art.

## Claims

1. A monoclonal antibody, or a fragment thereof comprising an antibody combining site, which binds specifically to an epitope in glycosylated native human albumin, which epitope comprises the glycosylated form of the lysine residue at position 525.

2. The monoclonal antibody or fragment thereof of claim 1 which binds specifically to a glycosylated peptide epitope of the formula:

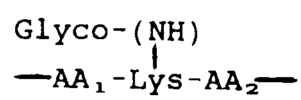

wherein Glyco-(NH) represents a nonenzymatically glycosylated ε-amino group in the lysine residue, and one or both of $AA_1$ and $AA_2$ is a sequence of amino acids wherein at least one of the amino acid units is in a position corresponding to the peptide sequence of human albumin adjacent to the lysine residue at position 525, and if only one of $AA_1$ and $AA_2$ is such a sequence, then the other is a bond, a terminal amino or carboxyl group, or additional amino acid residues.

3. The monoclonal antibody or fragment of claim 1 or 2 which binds specifically to said epitope in glycosylated native human albumin that has been treated by physical or chemical denaturation or digestion to expose said epitope for binding.

4. The monoclonal antibody or fragment of claim 3 wherein said epitope is exposed for binding by denaturation with a chaotropic agent.

5. An immunogen of the formula:

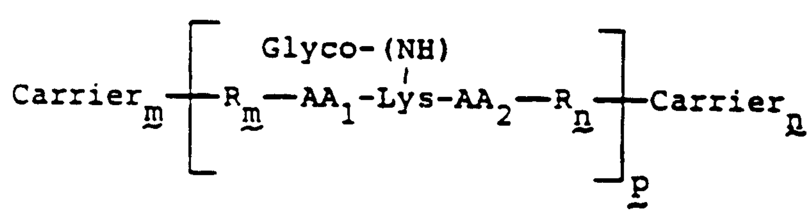

wherein Glyco-(NH) represents a nonenzymatically glycosylated ε-amino group in the lysine residue; one or both of $AA_1$ or $AA_2$ is a sequence of amino acids wherein at least one of the amino acid units is in a position corresponding to the peptide sequence of human albumin adjacent to the lysine residue at position 525, and if only one of $AA_1$ and $AA_2$ is such a sequence, then the other is a bond a terminal amino or carboxyl group, or additional amino acid residues; R is a bond or linking group; Carrier is an immunogenic carrier material; one of m and n is 1 and the other is zero; and p is on the average from 1 to the number of available coupling sites on Carrier.

**6.** An immunoassay method for determining glycosylated albumin in a human blood sample, comprising the steps of:

a) contacting the blood sample with a monoclonal antibody, or a fragment thereof comprising an antibody combining site, which binds specifically to an epitope in glycosylated native human albumin, which epitope comprises the glycosylated form of the lysine residue at position 525, and

(b) determining binding of the monoclonal antibody or fragment thereof to glycosylated human albumin as a function of the amount thereof in the sample tested.

**7.** The immunoassay method of claim 6 wherein said blood sample is treated to physically or chemically denature or digest glycosylated albumin therein to expose said epitope for binding, preferably by treatment with a chaotropic agent.

**8.** A peptide of the formula:

$$(NH_2)(Cys)_{\underset{\sim}{g}}-(Tyr)_{\underset{\sim}{r}}-AA_1-\overset{(QNH)}{\overset{|}{Lys}}-AA_2-(Tyr)_{\underset{\sim}{s}}-(Cys)_{\underset{\sim}{t}}(COOH)$$

wherein at least one of $AA_1$ and $AA_2$ is a sequence of from 1 to 12 amino acids corresponding to the peptide sequence adjacent to the lysine residue at position 525 in human albumin, and if only one of $AA_1$ and $AA_2$ is such a sequence, then the other is a bond, and wherein the N-terminal amino group in $(NH_2)AA_1$ and any lysine units in $AA_1$ or $AA_2$ can be glycosylated or non-glycosylated; wherein g and t are independently zero or 1; wherein r and s are, independently, zero, 1 or 2; wherein QNH represents the ε-amino group in Lys; and wherein Q is hydrogen or 1-deoxyfructosyl.

**9.** A peptide selected from the group comprising

a)

$$(NH_2)\overset{QNH}{\overset{|}{Lys}}-Gln-Thr-Ala-Leu-Val-Glu-Leu-Val-Lys(COOH)$$

wherein Q is 1-deoxyfructosyl;

b)

$$(NH_2)Arg-Gln-Ile-Lys-\overset{(QNH)}{\overset{|}{Lys}}-Gln-Thr-Ala-Leu-Val-$$
$$-Glu(COOH)$$

wherein Q is 1-deoxyfructosyl

c)

$$(NH_2)AA_3-Gln-Ile-\overset{*}{Lys}-\overset{(QNH)}{\overset{|}{Lys}}-Gln-Thr-Ala-Leu-Val-$$
$$-AA_4(COOH)$$

wherein Q is hydrogen or 1-deoxyfructosyl; $AA_3$ is Lŷs-Glu-Arg, Arg, or a bond; and $AA_4$ is Tyr-Cys or a bond; and wherein the N-terminal amino group in $(NH_2)AA_3$ and any Lŷs units in the peptide are glycosylated or non-glycosylated;

d)

(QNH)

$(NH_2)AA_4$-Glu-Arg-Gln-Ile-Ly̌s-Lys-Gln-Thr-Ala-
-Leu(COOH)

wherein Q is hydrogen or 1-deoxyfructosyl and $AA_4$ is Cys or a bond; and wherein the N-terminal amino group in $(NH_2)AA_4$ and the Ly̌s unit in the peptide are glycosylated or non-glycosylated;
e)

(QNH)

$(NH_2)$Gln-Ile-Ly̌s-Lys-Gln-Thr-Ala-Leu-Val-Glu-Leu-
-$AA_4$(COOH)

wherein Q is hydrogen or 1-deoxyfructosyl, $AA_4$ is Cys or a bond, and the N-terminal amino group in $(NH_2)$-Gln and the Ly̌s unit in the peptide are glycosylated or non-glycosylated;
f)

(QNH)

$(NH_2)AA_5$-Gln-Ile-Ly̌s-Lys-Gln-Thr-Ala-Leu-Val-
-Glu(COOH)

wherein Q is hydrogen or 1-deoxyfructosyl, $AA_5$ is Arg or a bond, and the N-terminal amino group on $(NH_2)AA_5$ and the Ly̌s unit in the peptide are glycosylated or non-glycosylated;
g)

(QNH)

$(NH_2)AA_6$-Lys-Gln-Thr-Ala-Leu-$AA_7$(COOH)

wherein Q is hydrogen or 1-deoxyfructosyl; $AA_6$, is Gln-Ile-Ly̌s, Ile-Ly̌s, Ly̌s, or a bond; $AA_7$ is Tyr-Tyr-Cys, Tyr-Cys, Cys, or a bond; and wherein the N-terminal amino group in $(NH_2)AA_6$ and Ly̌s units in the peptide are glycosylated or non-glycosylated; and
h)

(QNH)

$(NH_2)AA_8$-Arg-Gln-Ile-Ly̌s-Lys-Gln-Thr

wherein Q is hydrogen or 1-deoxyfructosyl; $AA_8$ is Cys-Tyr-Tyr, Cys-Tyr-Cys, or a bond; and the N-terminal amino group $(NH_2)AA_8$ and Ly̌s are glycosylated or non-glycosylated.

**10.** An immunoassay test kit comprising an antibody according to any of claims 1 to 4.

**Patentansprüche**

1. Monoklonaler Antikörper oder ein Fragment davon, enthaltend eine Antikörperbindungsstelle, die spezifisch an ein Epitop im glykosilierten nativen humanen Albumin bindet, wobei das Epitop die glykosilierte Form des Lysinrests an Position 525 umfaßt.

2. Monoklonaler Antikörper oder Fragment davon nach Anspruch 1, der spezifisch an ein glykosiliertes Peptidepitop der Formel

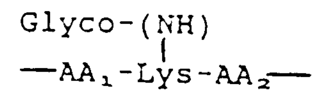

bindet, worin Glyko-(NH) eine nichtenzymatisch glykosilierte epsilon-Aminogruppe im Lysinrest darstellt und $AA_1$ und/oder $AA_2$ eine Sequenz von Aminosäuren ist, worin mindestens eine der Aminosäureeinheiten an einer Position entsprechend der Peptidsequenz des humanen Albumins benachbart zum Lysinrest an Position 525 ist, und falls nur $AA_1$ oder $AA_2$ eine solche Sequenz ist, ist die andere eine Bindung, eine terminale Amino- oder Carboxylgruppe oder zusätzliche Aminosäurereste.

3. Monoklonaler Antikörper oder Fragment nach Anspruch 1 oder 2, der spezifisch an das Epitop im glykosilierten nativen humanen Albumin bindet, das durch physikalische oder chemische Denaturierung oder Verdauung zur Freilegung des Epitops zur Antikörperbindung behandelt wurde.

4. Monoklonaler Antikörper oder Fragment nach Anspruch 3, worin das Epitop zur Bindung durch Denaturation mit einem chaotropen Agens freigelegt wird.

5. Immunogen der Formel

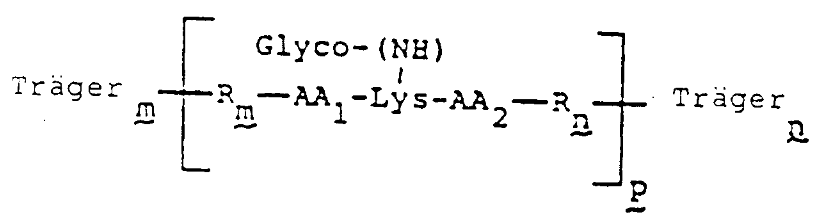

worin Glyko-(NH) eine nichtenzymatische glykosilierte epsilon-Aminogruppe im Lysinrest darstellt, $AA_1$ und/oder $AA_2$ ist eine Sequenz von Aminosäuren, worin mindestens eine Aminosäureeinheit in einer Position entsprechend der Peptidsequenz des humanen Albumin benachbart zum Lysinrest an Position 525 ist, und falls nur $AA_1$ oder $AA_2$ eine solche Sequenz ist, ist die andere eine Bindung oder eine terminale Amino- oder Carboxylgruppe oder zusätzlich Aminosäurereste; R ist eine Bindung oder eine Bindungsgruppe, der Träger ist ein immunogenes Trägermaterial, m ist 1 und n ist Null oder umgekehrt; und p ist im Mittel von 1 bis zur Anzahl der zugänglichen Kopplungsstellen auf dem Träger.

6. Immuntestverfahren zur Bestimmung von glykosiliertem Albumin in einer humanen Blutprobe, umfassend die Schritte:

a) Inkontaktbringen der Blutprobe mit einem monoklonalen Antikörper oder einem Fragment davon, welches eine Antikörperbindungsstelle enthält, die spezifisch an das Epitop im glykosilierten nativen humanen Albumin bindet, wobei das Epitop die glykosilierte Form des Lysinrests an Position 525 umfaßt, und

b) Bestimmen der Bindung des monoklonalen Antikörpers oder Fragments an das glykosilierte humane Albumin als Funktion seiner Menge in der zu testenden Probe.

7. Immuntestverfahren nach Anspruch 6, worin die Blutprobe physikalisch oder chemisch zur Denaturierung oder Verdauung glykosilierten Albumins behandelt wird, um darin das Epitop zur Bindung freizulegen, vorzugsweise durch Behandlung mit einem chaotropen Agens.

**8.** Peptid der Formel

$$\begin{array}{c} \text{(QNH)} \\ | \end{array}$$
$$(NH_2)(Cys)_q-(Tyr)_r-AA_1-Lys-AA_2-(Tyr)_s-(Cys)_t(COOH)$$

worin $AA_1$ und/oder $AA_2$ eine Sequenz von 1 bis 12 Aminosäuren entsprechend der Peptidsequenz, benachbart zum Lysinrest an Position 525 im humanen Albumin ist, und falls nur $AA_1$ oder $AA_2$ eine solche Sequenz ist, ist die andere eine Bindung, und worin die N-terminale Aminogruppe in $(NH_2)AA_1$ mit den Lysineinheiten in $AA_1$ oder $AA_2$ glykosiliert oder nichtglykosiliert sein können; worin q und t unabhängig voneinander Null oder 1 sind; worin r und s unabhängig voneinander Null, 1 oder 2 sind; worin QNH die epsilon-Aminogruppe im Lys darstellt, und worin Q Wasserstoff oder 1-Deoxyfructosyl ist.

**9.** Peptid, ausgewählt aus der Gruppe

a)

QNH
|
$(NH_2)$Lys-Gln-Thr-Ala-Leu-Val-Glu-Leu-Val-Lys(COOH)

worin Q 1-Deoxyfructosyl ist;

b)

(QNH)
|
$(NH_2)$Arg-Gln-Ile-Lys-Lys-Gln-Thr-Ala-Leu-Val-
-Glu(COOH)

worin Q 1-Deoxyfructosyl ist;

c)

(QNH)
* |
$(NH_2)AA_3$-Gln-Ile-Lys-Lys-Gln-Thr-Ala-Leu-Val-
$-AA_4$(COOH)

worin Q Wasserstoff oder 1-Deoxyfructosyl ist; $AA_3$ ist Lỹs-Glu-Arg, Arg, oder eine Bindung; und $AA_4$ ist Tyr-Cys oder eine Bindung; und worin die N-terminale Aminogruppe in $(NH_2)AA_3$ und die Lỹs-Einheiten im Peptid glykosiliert oder nichtglykosiliert sind;

d)

(QNH)
* |
$(NH_2)AA_4$-Glu-Arg-Gln-Ile-Lys-Lys-Gln-Thr-Ala-
-Leu(COOH)

worin Q Wasserstoff oder 1-Deoxyfructosyl ist und $AA_4$ Cys oder eine Bindung ist; und worin die N-terminale Aminogruppe $(NH_2)AA_4$ und die Lỹs-Einheit im Peptid glykosiliert oder nichtglykosiliert

sind;

e)

```
                                (QNH)
                          *       |
(NH2)Gln-Ile-Lys-Lys-Gln-Thr-Ala-Leu-Val-Glu-Leu-
-AA4(COOH)
```

worin Q Wasserstoff oder 1-Deoxyfructosyl ist, $AA_4$ ist Cys oder eine Bindung, und die N-terminale Aminogruppe im $(NH_2)$Gln und die Lŷs-Einheit im Peptid glykosiliert oder nichtglykosiliert sind;

f)

```
                                     (QNH)
                               *       |
(NH2)AA5-Gln-Ile-Lys-Lys-Gln-Thr-Ala-Leu-Val-
-Glu(COOH)
```

worin Q Wasserstoff oder 1-Deoxyfructosyl ist, $AA_5$ ist Arg oder eine Bindung, und die N-terminale Aminogruppe auf dem $(NH_2)AA_5$ und die Lŷs-Einheit im Peptid sind glykosiliert oder nichtglykosiliert;

g)

```
          (QNH)
            |
(NH2)AA6-Lys-Gln-Thr-Ala-Leu-AA7(COOH)
```

worin Q Wasserstoff oder 1-Deoxyfructosyl ist, $AA_6$ ist Gln-Ile-Lŷs, Ile-Lŷs, Lŷs, oder eine Bindung; $AA_7$ ist Tyr-Tyr-Cys, Tyr-Cys, Cys oder eine Bindung; und worin die N-terminale Aminogruppe im $(NH_2)AA_6$ und den Lŷs-Einheiten im Peptid glykosiliert oder nichtglykosiliert sind; und

h)

```
                                 (QNH)
                            *      |
(NH2)AA8-Arg-Gln-Ile-Lys-Lys-Gln-Thr
```

worin Q Wasserstoff oder 1-Deoxyfructosyl ist; $AA_8$ ist Cys-Tyr-Tyr, Cys-Tyr-Cys, oder eine Bindung; und die N-terminale Aminogruppe $(NH_2)AA_6$m und Lŷs sind glykosiliert oder nichtglykosiliert.

**10.** Immuntestkit, enthaltend einen Antikörper nach einem der Ansprüche 1 bis 4.

## Revendications

**1.** Anticorps monoclonal ou un fragment de ce dernier comprenant un site d'association à un anticorps, qui se lie spécifiquement à un déterminant antigénique présent dans l'albumine humaine naturelle glycosylée, ledit déterminant antigénique comprenant la forme glycosylée du résidu de lysine en position 525.

**2.** Anticorps monoclonal ou fragment de ce dernier selon la revendication 1, qui se lie spécifiquement à un déterminant antigénique peptidique glycosylé de formule

$$\begin{array}{l}\quad\text{Glyco-(NH)}\\ \qquad\quad |\\ \text{-AA}_1\text{-Lys-AA}_2\text{-}\end{array}$$

dans laquelle Glyco-(NH) représente un groupe ε-amino glycosylé par voie non enzymatique présent dans le résidu de lysine, et une des séquences $AA_1$ et $AA_2$ ou les deux représentent une séquence d'acides aminés, dans laquelle au moins une des unités d'acides aminés se trouve dans une position, correspondant à la séquence peptidique de l'albumine humaine, adjacente au résidu de lysine en position 525, et si seulement une des séquences $AA_1$ et $AA_2$ représente une telle séquence, alors l'autre représente une liaison, un groupe amino ou carboxyle terminal ou encore des résidus d'acides aminés supplémentaires.

**3.** Anticorps ou fragment monoclonal selon la revendication 1 ou 2 qui se lie spécifiquement audit déterminant antigénique présent dans l'albumine humaine naturelle glycosylée qui a été traitée par dénaturation ou digestion par voie chimique ou physique pour exposer ledit déterminant antigénique à des fins de liaison.

**4.** Anticorps ou fragment monoclonal selon la revendication 3, dans lequel on expose ledit déterminant antigénique à des fins de liaison, par dénaturation à l'aide d'un agent chaotrope.

**5.** Immunogène de formule

$$\text{support}_{\underline{m}}\left[\begin{array}{c}\text{Glyco-(NH)}\\ |\\ \text{R}_{\underline{m}}\text{—AA}_1\text{-Lys-AA}_2\text{—R}_{\underline{n}}\end{array}\right]_{\underline{p}}\text{support}_{\underline{n}}$$

dans laquelle Glyco-(NH) représente un groupe ε-amino glycosylé par voie non enzymatique présent dans le résidu de lysine; une des séquences $AA_1$ et $AA_2$ ou les deux représentent une séquence d'acides aminés dans laquelle au moins une des unités d'acides aminés se trouve dans une position, correspondant à la séquence peptidique de l'albumine humaine, adjacente au résidu de lysine en position 525, et si seulement une des séquences $AA_1$ et $AA_2$ représente une telle séquence, alors l'autre représente une liaison, un groupe amino ou carboxyle terminal ou encore des résidus d'acides aminés supplémentaires; R représente une liaison ou un groupe qui se lie; le terme "support" désigne une matière de support immunogène; un des indices $\underline{m}$ et $\underline{n}$ est égal à 1 et l'autre est égal à zéro; et $\underline{p}$ représente en moyenne de 1 site de couplage au nombre de sites de couplage disponibles sur le support.

**6.** Procédé d'immunodosage pour déterminer la teneur en albumine glycosylée dans un échantillon de sang humain, comprenant les étapes consistant à :

a) mettre l'échantillon de sang en contact avec un anticorps monoclonal ou un fragment de ce dernier comprenant un site d'association à un anticorps, qui se lie spécifiquement à un déterminant antigénique présent dans l'albumine humaine naturelle glycosylée, ledit déterminant antigénique comprenant la forme glycosylée du résidu de lysine en position 525, et

b) déterminer la liaison de l'anticorps monoclonal ou du fragment de ce dernier à l'albumine humaine glycosylée en fonction de la quantité de cette dernière dans l'échantillon soumis à l'essai.

**7.** Procédé d'immunodosage selon la revendication 6, dans lequel on traite ledit échantillon de sang pour dénaturer ou digérer par voie physique ou chimique l'albumine glycosylée qui y est contenue dans le but d'exposer ledit déterminant antigénique à des fins de liaison, de préférence par traitement avec un agent chaotrope.

**8.** Peptide de formule :

$$(NH_2)(Cys)_{\underset{\sim}{q}}-(Tyr)_{\underset{\sim}{r}}-AA_1-\overset{\overset{(QNH)}{|}}{Lys}-AA_2-(Tyr)_{\underset{\sim}{s}}-(Cys)_{\underset{\sim}{t}}(COOH)$$

dans laquelle au moins une des séquences $AA_1$ et $AA_2$ représente une séquence contenant de 1 à 12 acides aminés correspondant à la séquence peptidique adjacente au résidu de lysine en position 525 dans l'albumine humaine, et si seulement une des séquences $AA_1$ et $AA_2$ représente une telle séquence, alors l'autre représente une liaison; et dans laquelle le groupe amino N-terminal présent dans $(NH_2)AA_1$ et n'importe quelle unité de lysine dans $AA_1$ ou $AA_2$ peuvent être glycosylés ou non glycosylés; dans laquelle $\underset{\sim}{q}$ et $\underset{\sim}{t}$ représentent, de manière indépendante, zéro ou 1; et dans laquelle $\underset{\sim}{r}$ et $\underset{\sim}{s}$ représentent, de manière indépendante, zéro, 1 ou 2; dans laquelle QNH représente le groupe $\epsilon$-amino présent dans le résidu Lys; et dans laquelle Q représente un atome d'hydrogène ou un groupe 1-désoxyfructosyle.

**9.** Peptide choisi parmi le groupe comprenant

a)

$$(NH_2)\overset{\overset{QNH}{|}}{Lys}-Gln-Thr-Ala-Leu-Val-Glu-Leu-Val-Lys(COOH)$$

où Q représente un groupe 1-désoxyfructosyle.

b)

$$(NH_2)Arg-Gln-Ile-Lys-\overset{\overset{(QNH)}{|}}{Lys}-Gln-Thr-Ala-Leu-Val-$$
$$-Glu(COOH)$$

où Q représente un groupe 1-désoxyfructosyle.

c)

$$(NH_2)AA_3-Gln-Ile-\overset{*}{Lys}-\overset{\overset{(QNH)}{|}}{Lys}-Gln-Thr-Ala-Leu-Val-$$
$$-AA_4(COOH)$$

où Q représente un atome d'hydrogène ou un groupe 1-désoxyfructosyle; $AA_3$ représente $\overset{*}{Lys}$-Glu-Arg, Arg ou une liaison et $AA_4$ représente Tyr-Cys ou une liaison; et où le groupe amino N-terminal présent dans $(NH_2)AA_3$ et n'importe quelle unité $\overset{*}{Lys}$ présente dans le peptide sont glycosylés ou non glycosylés;

d)

$$(NH_2)AA_4-Glu-Arg-Gln-Ile-\overset{*}{Lys}-\overset{\overset{(QNH)}{|}}{Lys}-Gln-Thr-Ala-$$
$$-Leu(COOH)$$

où Q représente un atome d'hydrogène ou un groupe 1-désoxyfructosyle et $AA_4$ représente Cys ou une liaison et où le groupe amino N-terminal présent dans $(NH_2)AA_4$ et l'unité Lys* présente dans le peptide sont glycosylés ou non glycosylés;

e)

(QNH) on the second Lys:

$(NH_2)$Gln-Ile-Lys*-Lys(QNH)-Gln-Thr-Ala-Leu-Val-Glu-Leu-
-$AA_4$(COOH)

où Q représente un atome d'hydrogène ou un groupe 1-désoxyfructosyle, $AA_4$ représente Cys ou une liaison et le groupe amino N-terminal présent dans $(NH_2)$-Gln et l'unité Lys* présente dans le peptide sont glycosylés ou non glycosylés;

f)

$(NH_2)AA_5$-Gln-Ile-Lys*-Lys(QNH)-Gln-Thr-Ala-Leu-Val-
-Glu(COOH)

où Q représente un atome d'hydrogène ou un groupe 1-désoxyfructosyle, $AA_5$ représente Arg ou une liaison et le groupe amino N-terminal présent dans $(NH_2)AA_5$ et l'unité Lys* présente dans le peptide sont glycosylés ou non glycosylés;

g)

$(NH_2)AA_6$-Lys(QNH)-Gln-Thr-Ala-Leu-$AA_7$(COOH)

où Q représente un atome d'hydrogène ou un groupe 1-désoxyfructosyle; $AA_6$ représente Gln-Ile-Lys*, Ile-Lys*, Lys* ou une liaison; $AA_7$ représente Tyr-Tyr-Cys, Tyr-Cys, Cys ou une liaison et où le groupe amino N-terminal présent dans $(NH_2)AA_6$ et les unités Lys* présentes dans le peptide sont glycosylés ou non glycosylés;

h)

$(NH_2)AA_8$-Arg-Gln-Ile-Lys*-Lys(QNH)-Gln-Thr

où Q représente un atome d'hydrogène ou un groupe 1-désoxyfructosyle; $AA_8$ représente Cys-Tyr-Tyr; Cys-Tyr-Cys ou une liaison; et le groupe amino N-terminal présent dans $(NH_2)AA_8$ et Lys* sont glycosylés ou non glycosylés;

**10.** Nécessaire pour un immunodosage comprenant un anticorps selon l'une quelconque des revendications 1 à 4.

FIG. 1

GLYCOALBUMIN PEPTIDES

(1) --LYS-GLU-ARG-GLN-ILE-LYS-LYS-GLN-THR-ALA-LEU-VAL-GLU-LEU-VAL-LYS-HIS-LYS---
(525)
TRYPTIC PEPTIDE

(2) --LYS-GLU-ARG-GLN-ILE-LYS-LYS-GLN-THR-ALA-LEU-VAL-GLU-LEU-VAL-LYS-HIS-LYS---
(525)
V8 PEPTIDE

(3) *LYS-GLU-ARG-GLN-ILE-LYS-LYS-GLN-THR-ALA-LEU-VAL-(TYR-CYS)-COOH ALB K14C
(525)

(4) *(CYS)-GLU-ARG-GLN-ILE-LYS-LYS-GLN-THR-ALA-LEU-COOH ALB C11L
(525)

(5) *GLN-ILE-LYS-LYS-GLN-THR-ALA-LEU-VAL-GLU-LEU-(CYS)-COOH ALB Q12C
(525)

(6) *ARG-GLN-ILE-LYS-LYS-GLN-THR-ALA-LEU-VAL-GLU-COOH ALB R11E
(525)

(7) * LYS-GLN-THR-ALA-LEU-VAL-GLU-LEU-VAL-(CYS)-COOH ALB Q9C
(525)

FIG. 2

| | | |
|---|---|---|
| (8) | LYS*-GLN-THR-ALA-LEU-TYR-TYR-CYS<br>(525) | ALB Q7C<br>ALB K8C |
| (9) | LYS*-LYS*-GLN-THR-ALA-LEU-TYR-TYR-CYS<br>(525) | ALB K9C |
| (10) | *ILE-LYS*-LYS*-GLN-THR-ALA-LEU-TYR-TYR-CYS<br>(525) | ALB I10C |
| (11) | *GLN-ILE-LYS*-LYS*-GLN-THR-ALA-LEU-TYR-TYR-CYS<br>(525) | ALB Q11C |
| (12) | *CYS-TYR-TYR-ARG-GLN-ILE-LYS*-LYS*-GLN-THR<br>(525) | ALB C10T |